# EUROPÄISCHE PATENTSCHRIFT

(11) **EP 0 475 202 B1**
(45) Veröffentlichungstag und Bekanntmachung des Hinweises auf die Patenterteilung: **07.06.1995**
(21) Anmeldenummer: 91114497.0
(22) Anmeldetag: 29.08.1991
(51) Int. Cl.: A61F 13/15, B65H 45/16

(54) **Vorrichtung zum Falten von Hygieneprodukten**
Device for folding hygienic products
Dispositif pour plier des produits hygiéniques

(30) Priorität: 12.09.1990 DE 4028889
(43) Veröffentlichungstag der Anmeldung: 18.03.1992
(73) Patentinhaber: WINKLER & DÜNNEBIER MASCHINENFABRIK UND EISENGIESSEREI KG, D-56564 Neuwied (DE)
(72) Erfinder: Munsch, Klaus, Dipl.-Ing. (FH), W-5450 Neuwied 22 (DE)
(74) Vertreter: Schieferdecker, Lutz, Dipl.-Ing.

(56) Entgegenhaltungen:
- EP-A- 0 229 004
- FR-A- 412 368
- GB-A- 535 717
- US-A- 1 481 252
- US-A- 2 981 540
- US-A- 3 517 920
- US-A- 3 685 820
- US-A- 3 994 486

## Beschreibung

Die Erfindung betrifft eine Vorrichtung zum Falten von Hygieneprodukten und insbesondere von weichen, länglichen Teilen geringer Dicke wie beispielsweise von Windelhöschen usw. und umfaßt Merkmale gemäß dem Oberbegriff des Patentanspruches 1.

Eine Vorrichtung der genannten Art ist aus der US-A-3,337, 211 bekannt und umfaßt Faltstößel, die an der Faltstelle auf das sich bewegende, längliche Teil auftreffen und es quer zu seiner Bewegungsrichtung faltend in Stößelrichtung in eine Aufnahme bzw. in einen Spalt verschieben. Ferner ist ein Getriebe in Gestalt eines Zahnradgetriebes als Antrieb für den Faltstößel vorgesehen und umfaßt ein stillstehendes Sonnenrad, das auf einer Achse zwischen Gestellwänden sowie im Inneren einer auf der Achse drehbar gelagerten Umlenk- und Falttrommel gelagert ist. Das als Antrieb dienende Zahnradgetriebe umfaßt ferner ein Zwischenrad und ein den Stößel steuerndes Planetenrad. Ferner sind Kettenräder an beiden Enden der Umlenk- und Falttrommel vorgesehen und setzen diese in Drehung.

Gelöst wird die der Erfindung zugrundeliegende Aufgabe mit Hilfe der im kennzeichnenden Teil des Anspruches 1 enthaltenen Merkmale.

Das stillstehende Sonnenrad ist nicht auf einer die Umlenk- und Falttrommel lagernden Achse angeordnet, sondern an einer Gestellwand und der Faltstößel wird nicht mit Hilfe der sich drehenden Umlenk- und Falttrommel relativ zu dem stehenden Sonnenrad bewegt, sondern mit Hilfe eines eigenen Antriebes und einer Welle, die ebenfalls in der Gestellwand drehbar gelagert ist. Der Faltstößel weist somit einen direkten und unmittelbaren Antrieb und nicht einen indirekten Antrieb wie bei der Vorrichtung nach der US-A-3,337,211 auf. Der Antrieb und die Lagerung der für die Bewegung des Faltstößels erforderlichen Getriebeteile ist einfacher und weniger aufwendig als bisher und gestatten somit höhere Drehzahlen.

Weitere Merkmale der Erfindung gehen aus Unteransprüchen im Zusammenhang mit der Beschreibung und der Zeichnung hervor.

Die Erfindung wird nachstehend anhand eines Ausführungsbeispieles, das in der Zeichnung dargestellt ist, näher beschrieben. Dabei zeigen:
Die Arbeitsgeschwindigkeit der bekannten Faltvorrichtungen ist grundsätzlich hoch, sie genügt aber dennoch nicht in allen Fällen den Anforderungen. Insbesondere da die übrigen Komponenten der Produktionsmaschinen mit höheren Stundenleistungen betrieben werden könnten, stellen die Faltvorrichtungen einen Engpaß dar, der die Arbeitsgeschwindigkeit der Gesamtanlage entscheidend beeinflußt.

Der Erfindung liegt daher die Aufgabe zugrunde, eine Vorrichtung zum Falten von Hygieneartikeln anzugeben, die es erlaubt, die Arbeitsgeschwindigkeit zu erhöhen.
- Fig. 1:: zum Teil im Schnitt eine Prinzipskizze von wesentlichen Teilen der Vorrichtung zum Falten in Seitenansicht;
- Fig. 2:: eine Prinzipskizze in kleinerem Maßstab sowie in Stirnansicht von zwei nachgeschalteten Vorrichtungen gemäß Fig. 1;
- Fig. 3:: eine Einzelheit aus Fig. 2 in größerem Maßstab;
- Fig. 4:: eine abgebrochene Ansicht der Abwicklung des Trommelumfanges gem. Fig. 3;
- Fig. 5:: eine Bewegungsstudie eines symmetrisch wirksamen Faltstößels;
- Fig. 6:: eine Bewegungsstudie eines asymmetrisch wirksamen Faltstößels;
- Fig. 7:: eine Wiedergabe des Bewegungsablaufes des Faltstößels bei dem Umlauf eines ihn steuernden Planetenrades um ein Sonnenrad;
Eine Vorrichtung 1 zum Falten von Hygieneprodukten, insbesondere von weichen, länglichen Teilen 2 geringer Dicke, die an der vorgesehenen Faltstelle 3 mit Druck beaufschlagt und mit der Druckstelle voreilend sodann unter Faltung in eine Aufnahme bzw. in einen Spalt 4 verschoben werden, umfaßt einen Faltstößel 5 und einen Antrieb für den Faltstößel 5 in Gestalt eines Getriebes 6 sowie um Umlenkrollen laufende Transportbänder 7 und 8 zur Bildung der spaltartigen Aufnahme 4.

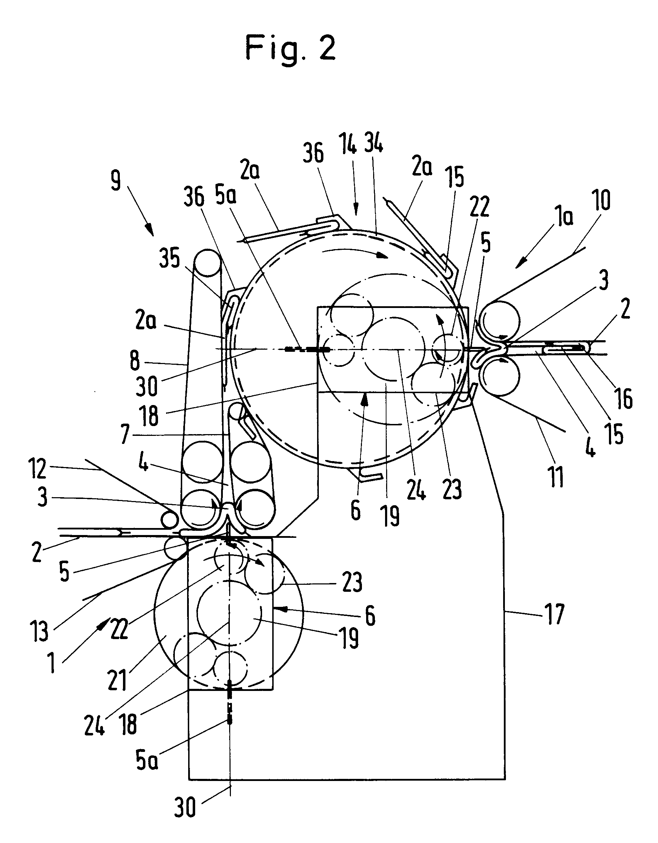

Gemäß Fig. 2 gehören zu einer Faltstation 9 zwei Vorrichtungen 1 und 1a. Auch die zweite Vorrichtung 1a weist zur Bildung des Spaltes 4 zwei um Umlenkrollen laufende Transportbänder 10 und 11 auf. Weitere Transportbänder 12 und 13 sind vorgesehen, um das zu faltende Teil 2 zunächst der ersten Vorrichtung 1 zuzuführen, ehe die senkrecht dazu den ersten Aufnahmespalt 4 bildenden Transportbänder 7 und 8 das einmal gefaltete Teil 2a einer Umlenk- und Falttrommel 14 der zweiten Faltvorrichtung 1a zuführen. Dort erfolgt die zweite Faltung, so daß sich die Enden 15 und 16 des Teiles 2a aufeinanderlegen.

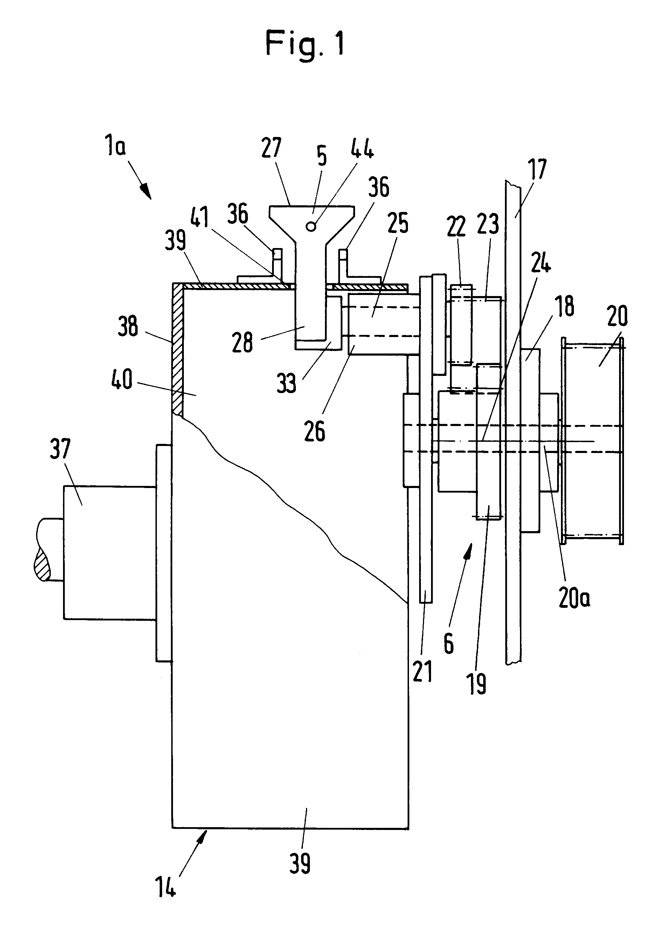

Der Antrieb für die beiden Vorrichtungen 1 und 1a gemäß den Figuren 1 und 2 ist ein Zahnradgetriebe in Form eines planetengetriebeartigen Getriebes 6. Es umfaßt ein an einer Gestellwand 17 mit Hilfe einer verstellbaren Trägerplatte 18 einstellbares, stillstehendes Sonnenrad 19 und einen auf einer ebenfalls in der Gestellwand 17 drehbar gelagerten Welle 20a angeordneten und von einem Antriebsrad 20 angetriebenen Träger 21 für ein Planetenrad 22 und für ein Zwischenrad 23, das einerseits mit dem Sonnenrad 19 und andererseits mit Planetenrad 22 kämmt. Der Träger 21 kann scheibenförmig sein.

Gemäß einer bevorzugten Ausführungsform verhält sich die Zähnezahl des Planetenrades 22 zur Zähnezahl des Sonnenrades 19 wie 1 : 2. Die Zähnezahl des Zwischenrades 23 ist beliebig. Wenn daher das Antriebsrad 20 über die Welle 20a den Träger 21 in Drehung versetzt, so umkreisen das Planetenrad 22 und das Zwischenrad 23 das stillstehende Sonnenrad 19, wobei ferner das Planetenrad 22 bei einer Rechtsdrehung des Trägers 21 in entgegengesetzter Richtung eine Linksdrehung ausführt.

Gemäß den Darstellungen in den Figuren 1 und 2 ist das nicht mit dem Sonnenrad 19 kämmende Planetenrad 22 zum Sonnenrad 19 axial sowie bezogen auf die Umlaufbahn seitlich neben dem Zwischenrad 23 versetzt angeordnet. Das mit dem Planetenrad 22 und dem Sonnenrad 19 kämmende Zwischenrad 23 besitzt eine Breite, die gleich der Breite von Sonnenrad 19 und Planetenrad 22 ist. Dadurch ist es möglich, das Planetenrad 22 und das Zwischenrad 23 nahe bei der Welle 20a bzw. bei der Achse 24 des Sonnenrades 19 anzuordnen.

An dem Träger 21 ist der Faltstößel 5 um einen Arm 25 frei drehbar gelagert und wird von dem Planetenrad 22 gesteuert. Als Arm 25 dient eine im Träger 21 drehbar gelagerte Welle, die das Planetenrad 22 mit dem Faltstößel 5 verbindet und die Drehbewegung des Planetenrades 22 auf den Faltstößel 5 überträgt. Zur Lagerung der Welle 25 mit dem Planetenrad 22 und dem Faltstößel 5 an dem Träger 21 dient eine Buchse 26.

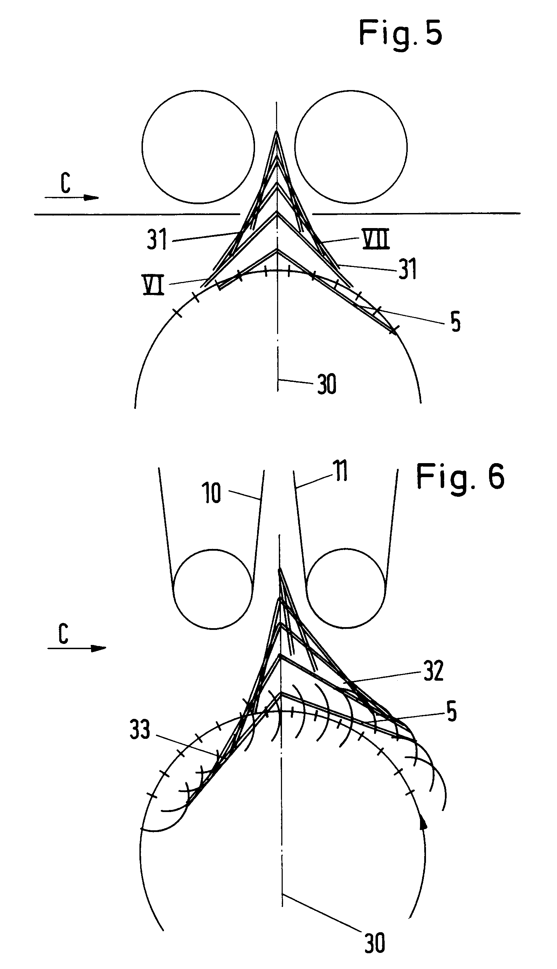

Die durch das Sonnenrad 19, das Planetenrad 22 und das Zwischenrad 23 erzeugten Bewegungen des Faltstößels 5, seines freien Endes 27 und seines anderen, wellenseitigen Endes 28 gehen aus den Darstellungen in den Figuren 5, 6 und 7 hervor. Dabei gilt folgendes.

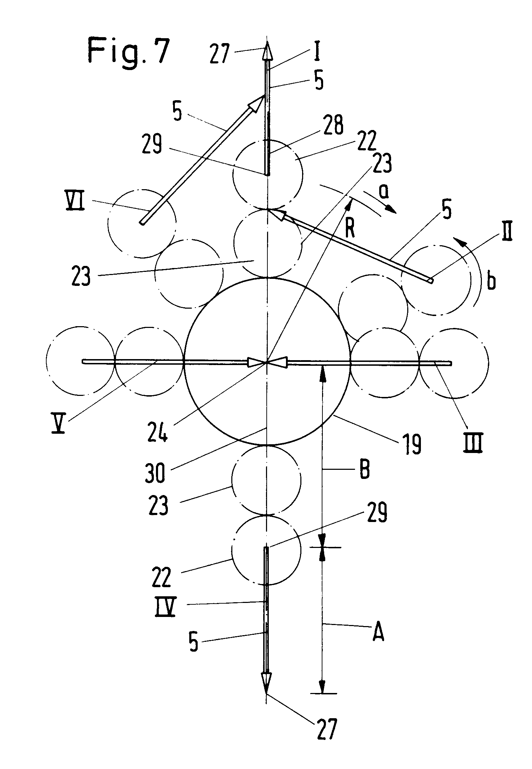

Wenn sich gemäß der Darstellung in Figur 7 das Planetenrad 22 mit seiner Drehachse 29 im Uhrzeigersinn auf einem Radius R in Richtung des Pfeiles a um die Achse 24 des Sonnenrades 19 bewegt, so dreht sich das Planetenrad 22 wegen des Zwischenrades 23 entgegen dem Uhrzeigersinn in Richtung des Pfeiles b. Aufgrund des vorzugsweise verwendeten Übersetzungsverhältnisses von 2 : 1 zwischen Sonnenrad 19 und Planetenrad 22 dreht sich das Planetenrad 22 um den gleichen Winkelbetrag entgegen dem Uhrzeigersinn, um den es im Uhrzeigersinn um das Sonnenrad wandert. Dies hat zur Folge, daß ein vom Planetenrad 22 gesteuerter Faltstößel 5 bei einem vollen Umlauf des Planetenrades 22 eine volle Linksdrehung ausführt, der zusätzlich die Umlaufbewegung des Planetenrades 22 überlagert ist. Dadurch kippt der Faltstößel 5 aus der Stellung I entsprechend seiner höchsten Faltposition beim Zurückziehen zunächst nach links entsprechend der Stellung II und dann weiter in die Stellung III gemäß Fig. 7, bis er die am weitesten zurückgezogene Stellung IV erreicht, wobei dort das freie Ende 27 des Faltstößels 5 in die entgegengesetzte Richtung zu der der Stellung I weist. Bei dem weiteren Umlauf des Planetenrades 22 hebt sich der Faltstößel 5 sodann wieder, durchläuft die Stellung V und trifft etwa in der Stellung VI auf das zu faltende Teil 2. Der Stellung VI in Fig. 7 entspricht beim Zurückziehen des Faltstößels aus der Stellung I eine spiegelbildlich rechts zur Bewegungsachse 30 liegende, in Fig. 7 nicht dargestellte Stellung.

Der Faltstößel 5 definiert sowohl selbst als auch mit seinem freien Ende 27 und seinem wellenseitigen Ende 28 im Bereich der Faltstelle 3 bei der Vorwärtsbewegung und bei der Rückwärtsbewegung eine Hüllkurve 31, die in Fig. 5 teilweise dargestellt ist. Diese Hüllkurve 31 läßt erkennen, daß der Faltstößel 5 nicht nur quer zur Transportrichtung c des zu faltenden Teiles 2, sondern auch in Transportrichtung c wechselnde Kräfte auf das Teil 2 ausübt, weil er sich aus der Stellung VI links zu Beginn des Faltvorganges bis in die Stellung VII (Fig. 5) rechts von der Bewegungsachse 30 am Ende des Faltvorganges bewegt.

Die Hüllkurve 31 in Fig. 5 gibt den Bewegungsablauf bei einem zur Bewegungsachse 30 symmetrisch wirksamen Faltstößel 5 wieder. Fig. 6 zeigt eine Hüllkurve 32 eines asymmetrisch wirksamen Faltstößels 5. Die asymmetrische Hüllkurve 32 wird erreicht, wenn der Faltstößel 5 an dem ihn tragenden Arm 25 nicht mittig, sondern außermittig angeordnet ist.

Eine symmetrisch wirksame Anordnung des Faltstößels 5 mit einer Hüllkurve 31 gemäß Fig. 5 ist vorteilhaft, wenn der Faltstößel 5 eine erste Faltung an einem langgestreckten Teil 2 gem. Fig. 2 in der ersten Vorrichtung 1 der Faltstation 9 ausführt. Die asymmetrisch wirksame Hüllkurve 32 und die außermittige Anordnung des Faltstößels 5 eignen sich vor allem bei der Herstellung der zweiten Faltung in der Vorrichtung 1a der Faltstation 9, denn hier soll der Faltstößel 5 nicht nur das bereits einmal gefaltete Teil 2a in die spaltförmige Aufnahme 3 drücken, sondern dabei auch noch verhindern, daß sich das bereits vorgefaltete Ende 15 wieder öffnet und beim Zurückziehen des Faltstößels 5 aus dem zusammengelegten Teil 2 herausgerissen wird.

Der Faltstößel 5 kann mit seinem wellenseitigen Ende 28 an der Welle 25 mit Hilfe eines Zwischenstückes 33 entweder symmetrisch gemäß Fig. 5 befestigt sein, oder gemäß Fig. 6 eine voreilende Position einnehmen und auch gemäß einer nicht dargestellten Befestigungsform eine nacheilende Position erhalten. Bei der voreilenden Position gem. Fig. 6 erreicht der Faltstößel 5 bereits die Stellung I in Fig. 7, noch bevor die Drehachse 29 des Planetenrades 22 die Bewegungsachse 30 schneidet. Bei einer nacheilenden Position ist der Faltstößel 5 an der Welle 25 derart exzentrisch angeordnet, daß die Drehachse 29 des Planetenrades 22 und der Welle 25 voreilt und die Bewegungsachse 30 bereits überschritten hat, bevor der Faltstößel 5 eine fluchtende Lage mit der Bewegungsachse 30 oder eine dazu parallele Stellung erreicht. Der Faltstößel 5 kann daher derart eingestellt werden, daß er entsprechend den jeweiligen Erfordernissen optimale Bewegungen ausführt.

Wie auch in Fig. 7 dargestellt ist, wird es als besonders zweckmäßig angesehen, wenn der Abstand A von der Drehachse 29 des Planetenrades 22 bzw. der Welle 25 bis zum freien Ende 27 des Faltstößels 5 ebenso groß ist wie der Abstand B von der Drehachse 29 des Planetenrades 22 bis zur Achse 24 des Sonnenrades 19. Dann bewegt sich nämlich das freie Ende 27 des Faltstößels 5 bei symmetrischer Anordnung zur Achse 29 längs der Bewegungsachse 30, während das Planetenrad 22 einen Umlauf durchführt.

Die Vorrichtung 1a in der Faltstation 9 gemäß Fig. 2 weist gegenüber der Vorrichtung 1 zusätzlich eine Umlenk- und Falttrommel 14 auf, auf deren Umfang 34 das von der Vorrichtung 1 kommende Teil 2a mit seinem Falz 35 voreilend aufläuft. Zum Halten der Teile 2a weist die Umlenk- und Falttrommel 14 in Umfangsrichtung versetzt paarweise angeordnete fingerartige Halteelemente 36 auf. Die Transportbänder 7 und 8 schieben die Teile 2a jeweils zwischen die fingerartigen Halteelemente 36 und den Umfang 34 der Umlenk- und Falttrommel 14.

Die Umlenk- und Falttrommel 14 sitzt frei auskragend auf einer Antriebswelle 37 und besteht aus einem Trommelboden 38 und einer zylindrischen Wand 39, die zum Getriebe 6 hin (Fig. 1) offen ist.

Der den Faltstößel 5 tragende Arm 25 bzw. die Welle 25 und auch die Buchse 26, die an dem zum Getriebe 6 gehörenden Träger 21 angeordnet ist, greifen von der offenen Seite her in das Innere 40 der Umlenk- und Falttrommel 14. Der Faltstößel 5 erstreckt sich rechtwinklig von dem Arm 25 weg bzw. steht radial oder im wesentlichen radial zur Drehachse 29 des Planetenrades 22.

Nahezu die gesamte Bewegung, die der Faltstößel 5 gemäß Fig. 7 durchführt, erfolgt im Inneren 40 der Umlenk- und Falttrommel 14. Nur für den unmittelbaren Faltvorgang greift der Faltstößel 5 mit seinem freien Ende 27 durch Durchtrittsöffnungen 41, während sein anderes Ende 28 ebenso wie der Arm 25 im Inneren 40 der Umlenk- und Falttrommel 14 verbleiben.

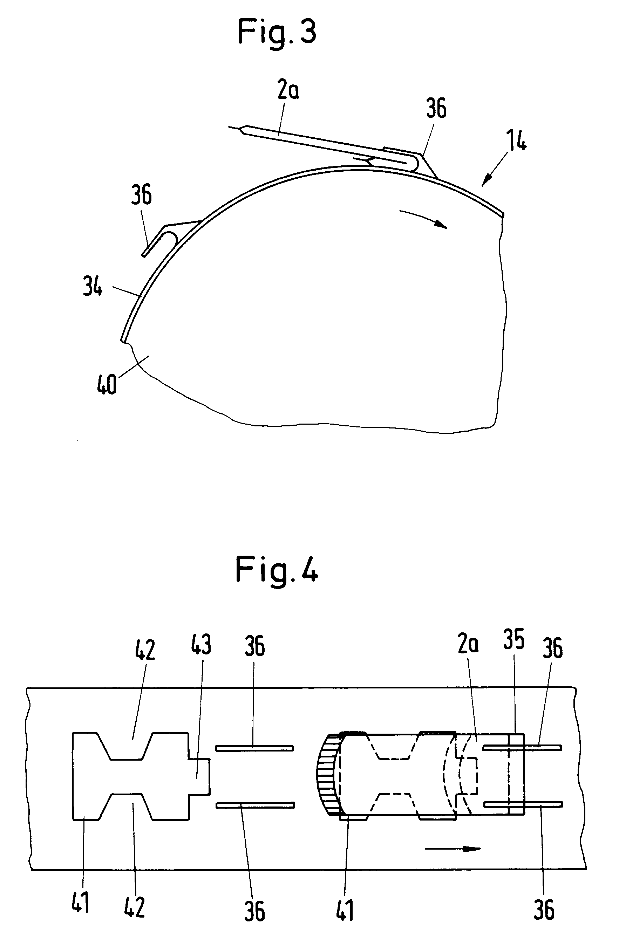

Je eine Durchtrittsöffnung 41 ist am Umfang 34 der Umlenk- und Falttrommel 14 jeder Gruppe von fingerartigen Halteelementen 36 zugeordnet. Wie Fig. 4 zeigt, befindet sich jeweils eine Durchtrittsöffnung 41 in Laufrichtung hinter den das einmal gefaltete Teil 2a haltenden fingerartigen Halteelementen 36. Die Durchtrittsöffnungen 41 weisen auf halber Länge einander zugewandte Vorsprünge 42 und eine den fingerartigen Halteelementen 36 zugewandte Ausnehmung 43 auf, die in Anbetracht der T-Form des Faltstößels 5 (Fig. 1) und der von ihm durchgeführten Bewegungen entsprechend den Hüllkurven 31 und 32 entsprechend den Figuren 5 und 6 zu der geringstmöglichen Öffnungsfläche der Durchtrittsöffnung 41 führen. Eine geringe Öffnungsfläche ist erwünscht, um den Eintritt von Staub und dgl. in das Innere 40 der Umlenk- und Falttrommel 14 zu vermeiden.

Im Betrieb arbeitet die Vorrichtung 1 mit ihrem Faltstößel 5 senkrecht nach oben, so daß sich das freie Ende 27 des Faltstößels 5 längs der in Fig. 2 senkrecht stehenden Bewegungsachse 30 bewegt. Bei der Vorrichtung 1a arbeitet der Faltstößel 5 horizontal und bewegt sich mit seinem freien Ende längs der horizontal liegenden Bewegungsachse 30. Nur gestrichelt dargestellt und mit 5a bezeichnet ist der Faltstößel zusammen mit dem Planetenrad und dem Zwischenrad in Fig. 2 in der Position IV gem. Fig. 7, also bei Erreichen einer halben Umlaufbahn, wenn sich das freie Ende 27 des Faltstößels 5 am weitesten von der Faltstelle 3 wegbewegt hat.

Die Umlenk- und Falttrommel 14 ist ein den Faltstößel 5 und wesentliche Teile seines Antriebes aufnehmendes und schützendes Gehäuse.

Um am freien Ende 27 des Faltstößels 5 nicht nur in Richtung der Bewegungsachse 30 wirkende Druckkräfte zu erhalten, kann ferner zur Unterstützung auch noch Druckluft seitlich am freien Ende 27 wirksam sein. Zu diesem Zweck befindet sich eine Blasdüse 44 seitlich am freien Ende 27 des Faltstößels 5 und ist in geeigneter, in den Figuren nicht dargestellter Weise mit einem Drucklufterzeuger und einer Steuereinrichtung verbunden.

Schließlich kann auch ein federndes Druckstück seitlich am freien Ende 27 des Faltstößels 5 angeordnet sein, um die erwünschten, seitlichen Drücke und Kräfte zu erzeugen.

## Patentansprüche

1. Vorrichtung zum Falten von Hygieneprodukten, insbesondere von weichen, länglichen Teilen (2) geringer Dicke, wie z.B. von Windelhöschen, Damenbinden etc., wobei ein Faltstößel (5, 5a) vorgesehen ist und an der Faltstelle (3) auf das sich bewegende, längliche Teil (2) auftrifft und es quer zu seiner Bewegungsrichtung faltend in Stößelrichtung in eine Aufnahme (4) bzw. in einen Spalt (4) verschiebt, wobei ferner ein Getriebe (6) in Gestalt eines Zahnradgetriebes als Antrieb für den Faltstößel (5) vorgesehen ist und ein stillstehendes Sonnenrad (19), ein Zwischenrad (23) und ein den Faltstößel (5) steuerndes Planetenrad (22) aufweist, dadurch gekennzeichnet, daß das stillstehende Sonnenrad (19) an einer Gestellwand (17) angeordnet ist, und daß eine angetriebene Welle (20a) in der Gestellwand (17) drehbar gelagert ist und daß auf der Welle (20a) ein Träger (21) für das Planetenrad (22) und für das Zwischenrad (23) angeordnet sind, wobei das Zwischenrad einerseits mit dem Sonnenrad (19) und andererseits mit dem Planetenrad (22) kämmt, und daß der Faltstößel (5, 5a) um einen Arm (25) frei drehbar an dem Träger (21) gelagert ist, wobei er von dem Planetenrad (22) gesteuert wird.

2. Vorrichtung nach Anspruch 1, dadurch gekennzeichnet, daß als Arm (25) eine im Träger (21) drehbar gelagerte Welle dient.

3. Vorrichtung nach Anspruch 1 und 2, dadurch gekennzeichnet, daß ein Antriebsrad (20) als Antrieb für die Welle (20a) vorgesehen ist und daß der Träger (21) scheibenförmig ist.

4. Vorrichtung nach einem oder mehreren der vorhergehenden Ansprüche, dadurch gekennzeichnet, daß sich die Zähnezahl des Planetenrades (22) zur Zähnezahl des Sonnenrades (19) wie 1 : 2 verhält.

5. Vorrichtung nach einem oder mehreren der vorhergehenden Ansprüche, dadurch gekennzeichnet, daß das nicht mit dem Sonnenrad (19) kämmende Planetenrad (22) zum Sonnenrad (19) axial sowie bezogen auf die Umlaufbahn seitlich neben dem Zwischenrad (23) versetzt angeordnet ist, wobei das mit dem Planetenrad (22) und dem Sonnenrad (19) kämmende Zwischenrad (23) eine Breite besitzt, die gleich der Breite von Sonnenrad (19) und Planetenrad (22) ist.

6. Vorrichtung nach einem oder mehreren der vorhergehenden Ansprüche, dadurch gekennzeichnet, daß der Abstand (A) von der Drehachse (29) des Planetenrades (22) bzw. der Welle (25) bis zum freien Ende (27) des Faltstößels (5) ebenso groß ist, wie der Abstand (B) von der Drehachse (29) des Planetenrades (22) bis zur Achse (24) des Sonnenrades (19).

7. Vorrichtung nach einem oder mehreren der vorhergehenden Ansprüche, dadurch gekennzeichnet, daß eine Umlenk- und Falttrommel (14) frei auskragend auf einer weiteren Antriebswelle (37) angeordnet ist, zum Getriebe (6) hin offen ist und dieses umgreift.

8. Vorrichtung nach einem oder mehreren der vorhergehenden Ansprüche, dadurch gekennzeichnet, daß die Umlenk- und Falttrommel aus einem Trommelboden (38) und einer zylindrischen Wand (39) besteht, die mindestens eine Durchtrittsöffnung (41) für den mit seinem freien Ende (27) hindurchgreifenden Faltstößel (5) aufweist.

9. Vorrichtung nach einem oder mehreren der vorhergehenden Ansprüche, dadurch gekennzeichnet, daß fingerartige Halteelemente (36) am Umfang (34) der Umlenk- und und Falttrommel (14) angeordnet sind.

10. Vorrichtung nach einem oder mehreren der vorhergehenden Ansprüche, dadurch gekennzeichnet, daß sich jeweils eine Durchtrittsöffnung (41 ) in Laufrichtung hinter den das Teil (2a) haltenden fingerartigen Halteelementen (36) befindet.

11. Vorrichtung nach einem oder mehreren der vorhergehenden Ansprüche, dadurch gekennzeichnet, daß der Faltstößel (5 ) T-förmig ist.

12. Vorrichtung nach einem oder mehreren der vorhergehenden Ansprüche, dadurch gekennzeichnet, daß eine Blasdüse (44) für Druckluft am freien Ende (27) des Faltstößels (5) angeordnet ist.

13. Vorrichtung nach einem oder mehreren der vorhergehenden Ansprüche, dadurch gekennzeichnet, daß das stillstehende Sonnenrad (19) einstellbar mit Hilfe einer verstellbaren Trägerplatte (18) an der Gestellwand (17) angeordnet ist.

14. Vorrichtung nach einem oder mehreren der vorhergehenden Ansprüche, dadurch gekennzeichnet, daß der Faltstößel (5) an dem ihn tragenden Arm (25) außermittig angeordnet ist.

15. Vorrichtung nach einem oder mehreren der vorhergehenden Ansprüche, dadurch gekennzeichnet, daß zum Falten eines mehrfach gefalteten Teiles (2) eine erste Vorrichtung (1) mit ihrem Faltstößel (5) senkrecht nach oben arbeitend angeordnet ist, während sich der Faltstößel (5) einer zweiten Vorrichtung (1a) für den zweiten Faltvorgang horizontal bewegt.

## Claims

1. Apparatus for folding hygiene products, in particular soft elongate pieces (2) of small thickness such as for example nappies, sanitary towels etc, wherein there is provided a folding thrust member (5, 5a) which at the folding location (3) strikes against the moving elongate piece (2) and folding it transversely to its direction of movement displaces it in the direction of the thrust member into a receiving means (4) or into a gap (4), wherein there is also provided a transmission (6) in the form of a gear transmission as a drive for the folding thrust member (5), the transmission having a stationary sun gear (19), an intermediate gear (23) and a planet gear (22) which controls the folding thrust member (5), characterised in that the stationary sun gear (19) is arranged on a support wall (17) and that a driven shaft (20a) is rotatably mounted in the support wall (17) and that a carrier (21) for the planet gear (22) and for the intermediate gear (23) is arranged on the shaft (20a), wherein the intermediate gear meshes on the one hand with the sun gear (19) and on the other hand with the planet gear (22), and that the folding thrust member (5, 5a) is mounted on the carrier (21) freely rotatably about an arm (25), the folding thrust member heing controlled by the planet gear (22).

2. Apparatus according to claim 1 characterised in that a shaft mounted rotatably in the carrier (21) serves as the arm (25).

3. Apparatus according to claim 1 and claim 2 characterised in that a drive wheel (20) is provided as the drive for the shaft (20a) and that the carrier (21) is of a disc-like configuration.

4. Apparatus according to one or more of the preceding claims characterised in that the number of teeth of the planet gear (22) to the number of teeth of the sun gear (19) is in a ratio of 1:2.

5. Apparatus according to one or more of the preceding claims characterised in that the planet gear (22) which does not mesh with the sun gear (19) is arranged displaced axially relative to the sun gear (19) and laterally beside the intermediate gear (23) with respect to the circular path of movement, wherein the intermediate gear (23) which meshes with the planet gear (22) and the sun gear (19) is of a width which is equal to the width of the sun gear (19) and the planet gear (22).

6. Apparatus according to one or more of the preceding claims characterised in that the distance (A) from the axis of rotation (29) of the planet gear (22) or the shaft (25) to the free end (27) of the folding thrust member (5) is the same as the distance (B) from the axis of rotation (29) of the planet gear (22) to the axis (24) of the sun gear (19).

7. Apparatus according to one or more of the preceding claims characterised in that a deflection and folding drum (14) is arranged in freely cantilever relationship on a further drive shaft (37), is open towards the transmission (6) and embraces same.

8. Apparatus according to one or more of the preceding claims characterised in that the deflection and folding drum comprises a drum end portion (38) and a cylindrical wall (39) which has at least one through opening (41) for the folding thrust member (5) which extends therethrough with its free end (27).

9. Apparatus according to one or more of the preceding claims characterised in that finger-like holding elements (36) are arranged at the periphery (34) of the deflection and folding drum (14).

10. Apparatus according to one or more of the preceding claims characterised in that a respective through opening (41) is disposed in the direction of travel behind the finger-like holding elements (36) for holding the piece (2a).

11. Apparatus according to one or more of the preceding claims characterised in that the folding thrust member (5) is T-shaped.

12. Apparatus according to one or more of the preceding claims characterised in that a blowing nozzle (44) for compressed air is arranged at the free end (27) of the folding thrust member (5).

13. Apparatus according to one or more of the preceding claims characterised in that the stationary sun gear (19) is arranged adjustably by means of a displaceable carrier plate (18) on the support wall (17).

14. Apparatus according to one or more of the preceding claims characterised in that the folding thrust member ( 5) is arranged eccentrically on the arm (25) carrying it.

15. Apparatus according to one or more of the preceding claims characterised in that for folding a repeatedly folded piece (2) a first apparatus (1) is arranged with its folding thrust member (5) operating perpendicularly upwardly while the folding thrust member (5) of a second apparatus (1a) moves horizontally for the second folding operation.

## Revendications

1. Dispositif pour le pliage de produits hygiéniques et en particulier de pièces (2) molles allongées et de faible épaisseur comme par exemple des couches-culottes, des serviettes hygiéniques etc., un poussoir de pliage (5, 5a) étant prévu qui, à la pliure (3), se dirige vers la pièce allongée (2) mobile et la pousse, transversalement à son sens de déplacement, dans un logement (4) respectivement dans une fente (4) en la pliant dans le sens du poussoir, de plus un engrenage (6) sous forme d'un engrenage à roue dentée étant prévu pour l entraînement du poussoir de pliage (5), engrenage qui présente une roue planétaire stationnaire (19), une roue intermédiaire (23) et une roue satellite (22) commandant le poussoir de pliage (5), caractérisé en ce que la roue planétaire stationnaire (19) est disposée contre une paroi de bâti (17), et en ce qu'un arbre mené (20a) est logé rotatif dans la paroi de bâti (17) et en ce que sur l'arbre (20a) est disposé un support (21) pour la roue satellite (22) et pour la roue intermédiaire (23), la roue intermédiaire venant en prise d'une part, avec la roue planétaire (19) et, d'autre part, avec la roue satellite (22), et en ce que le poussoir de pliage (5, 5a) est monté librement rotatif sur un support (21) autour d'un bras (25) et est commandé par la roue satellite (22).

2. Dispositif selon la revendication 1, caractérisé en ce qu'un arbre logé rotatif dans le support 21 fait office de bras (25).

3. Dispositif selon la revendication 1 et 2, caractérisé en ce qu'une roue menante (20) est prévue pour l'entraînement de l'arbre (20a) et en ce que le support (21) est en forme de disque.

4. Dispositif selon l'une ou plusieurs des revendications précédentes, caractérisé en ce que le rapport entre le nombre de dents de la roue satellite (22) et le nombre de dents de la roue planétaire (19) est de 1:2.

5. Dispositif selon l'une ou plusieurs des revendications précédentes caractérisé en ce que la roue satellite (22) qui n'est pas en prise avec la roue planétaire (19), est décalée latéralement à côté de la roue intermédiaire (23) et, ce, axialement par rapport à la roue planétaire (19) et à l'orbite, la roue intermédiaire (23), qui vient en prise avec la roue satellite (22) et avec la roue planétaire (19), présentant une largeur égale à la largeur de la roue planétaire (19) et de la roue satellite (22).

6. Dispositif selon l'une ou plusieurs des revendications précédentes, caractérisé en ce que la distance (A) entre l'axe de rotation (29) de la roue satellite (22) respectivement de l'arbre (25) et l'extrémité libre (27) du poussoir de pliage (5) est égale à la distance (B) entre l'axe de rotation (29) de la roue satellite (22) et l'axe (24) de la roue planétaire (19).

7. Dispositif selon l'une ou plusieurs des revendications précédentes caractérisé en ce qu'un tambour de pliage et de renvoi (14) est disposé en porte à faux sur un autre arbre menant (37) et s'ouvre sur l'engrenage (6) en entourant celui-ci.

8. Dispositif selon l'une ou plusieurs des revendications précédentes, caractérisé en ce que le tambour de pliage et de renvoi se compose d'un fond de tambour (38) et d'une paroi cylindrique (39) qui présente au moins une ouverture de passage (41) pour la traversée de l'extrémité libre (27) du poussoir de pliage (5).

9. Dispositif selon l'une ou plusieurs des revendications précédentes, caractérisé en ce que des éléments de retenue (36) en forme de doigts sont disposés sur la périphérie (34) du tambour de pliage et de renvoi (14).

10. Dispositif selon l'une ou plusieurs des revendications précédentes caractérisé en ce que chaque ouverture de passage (41) se situe dans le sens de la marche derrière les éléments de retenue (36) en forme de doigts maintenant la pièce (2a).

11. Dispositif selon l'une ou plusieurs des revendications précédentes, caractérisé en ce que le poussoir de pliage (5) présente la forme d'un T.

12. Dispositif selon l'une ou plusieurs des revendications précédentes, caractérisé en ce qu'une buse (44) de soufflage d'air comprimé est disposée à l'extrémité du poussoir de pliage (5).

13. Dispositif selon l'une ou plusieurs des revendications précédentes caractérisé en ce que la roue planétaire stationnaire (19) peut être disposée, de manière réglable, contre la paroi de bâti (17) à l'aide d'une plaque-support (18) ajustable.

14. Dispositif selon l'une ou plusieurs des revendications précédentes, caractérisé en ce que le poussoir de pliage (5) est disposé de manière excentrée sur son bras de support (25).

15. Dispositif selon l'une ou plusieurs des revendications précédentes, caractérisé en ce que, pour le pliage d'une pièce (2) pliée plusieurs fois, un premier dispositif (1) est disposé de manière à avoir son poussoir de pliage (5) travaillant à la verticale vers le haut, tandis que le poussoir de pliage (5) d'un second dispositif (1a) se déplace horizontalement pour la seconde opération de pliage.
